# EUROPEAN PATENT APPLICATION

(11) **EP 3 925 468 A1**
(43) Date of publication of application: **22.12.2021**
(21) Application number: 19922380.1
(22) Date of filing: 05.12.2019
(51) Int. Cl.: A24F 47/00, A61M 11/04

(54) **ATOMIZATION DEVICE CAPABLE OF CONTROLLING ADMINISTRATION AMOUNT, AND CONTROL METHOD FOR SAME**

(30) Priority: 04.04.2019 CN 201910269123
(71) Applicant: Huizhou Happy Vaping Technology Limited, Huizhou, Guangdong 516000 (CN)
(72) Inventor: LIN, Guangrong, Shenzhen, Guangdong 518104 (CN); ZHENG, Xianbin, Shenzhen, Guangdong 518104 (CN); ZHANG, Xiyong, Shenzhen, Guangdong 518104 (CN)
(74) Representative: Meyer, Thorsten
(86) International application number: PCT/CN2019/123198
(87) International publication number: WO 2020/199634

(57) **Abstract**

The disclosure provides a vaporizing device capable of controlling administration amount and a controlling method thereof. The vaporizing device comprises a battery (1), a control circuit board (2), a heating resistor (3), a vaporizing passage (4), a mouth piece (7), a detecting air passage (5), and an air pressure sensor (6). The control circuit board (2) is arranged with a microcontroller, an air flow calculating unit, an energy statistics unit, an energy and vaporizing amount conversion unit, and a power control unit, which are electrically connected with each other. The air flow calculating unit calculates corresponding air flow quantity based on the modeling volume of the detecting air passage and the change of suction pressure. The power control unit controls the power output to the heating resistor (3) based on the air flow quantity. The energy statistics unit calculates output energy based on the output power and the time. The energy and vaporizing amount conversion unit calculates corresponding value of aerosol intake from the energy. The microcontroller compares it with preset limit value of aerosol intake and then sends corresponding control signal to the power control unit to limit the aerosol intake.

## Description

### TECHNICAL FIELD

The disclosure relates to the field of vaporizing devices, more particularly to a vaporizing device capable of controlling administration amount and a method of controlling the vaporizing device capable of controlling administration amount.

### BACKGROUND

The vaporizing device usually heats and vaporizes liquid substances or paste-like substances, such as drugs and E-cigarette liquid, to generate aerosol or vapor for users to use. As people pay more attention to their health, they realize that overuse may be harmful to health.

Existing vaporizing devices usually can be freely used by the users without any appropriate restriction or control during using. The administration amount of vaporized aerosol or vapor cannot be calculated, controlled or restricted until the substances to be vaporized, such as drugs and E-cigarette liquid, run out. In such a case, the users may overuse after a long period of use and the too much intake probably will be harmful to health.

### SUMMARY

### Technical problem

An object of the disclosure is to overcome the above shortcomings and provide a vaporizing device capable of controlling administration amount. The vaporizing device capable of controlling administration amount may calculate, control or restrict the administration amount of the vaporized aerosol or vapor.

### Technical solutions

The disclosure provides a technical solution as follow. A vaporizing device capable of controlling administration amount comprises a battery, a control circuit board, a heating resistor, a vaporizing passage, and a mouth piece. The heating resistor is disposed in the vaporizing passage, and is controlled by means of the control circuit board to heat and vaporize liquid substances or paste-like substances, to generate aerosol or vapor for inhale. The vaporizing device capable of controlling administration amount further comprises a detecting air passage and an air pressure sensor disposed in the detecting air passage to detect the suction pressure. The control circuit board is arranged with a microcontroller, an air flow calculating unit, an energy statistics unit, an energy and vaporizing amount conversion unit, and a power control unit, which are electrically connected with each other. The air flow calculating unit is configured to calculate the corresponding air flow quantity based on the modeling volume of the detecting air passage and the change of suction pressure. The power control unit is configured to control output power output to the heating resistor based on the air flow quantity. The energy statistics unit is configured to calculate the energy output to the heating resistor based on the output power and the time. The energy and vaporizing amount conversion unit is configured to calculate the corresponding value of aerosol intake from the energy. The microcontroller is configured to compare the value of aerosol intake with preset limit value of aerosol intake and then send corresponding control signal to the power control unit. On such basis, the power control unit is configured to control the output power to limit the aerosol intake.

Preferably, the limit value of aerosol intake set in the microcontroller may comprise the limit value of single puff intake. The power control unit may be configured to shut down the output if the value of single puff intake exceeds the limit value of single puff intake.

Preferably, the limit value of aerosol intake set in the microcontroller may further comprise the limit value of total intake. The power control unit may be configured to shut down the output if the sum value of aerosol intake per unit time exceeds the limit value of total intake.

Preferably, the control circuit board may be further arranged with a resistance detection unit. The resistance detection unit is configured to detect a resistance change of the heating resistor and send the value of the resistance change to the microcontroller, to allow the microcontroller to control the power control unit based on the value of the resistance change.

Preferably, the control circuit board may be further arranged with a real-time clock unit and a timing unit.

Preferably, the control circuit board may comprise a power on unit for turning on and activating the vaporizing device.

Preferably, a warning unit electrically connected with the microcontroller may be further provided.

Preferably, the control circuit board may be further arranged with a wireless communication module electrically connected with the microcontroller. The wireless communication module may be in communication with the wireless communication module of the APP. By wireless communication, the APP is configured to read the information of the vaporizing device and control operations of the vaporizing device.

Preferably, the APP may comprise a setting interface for a limit value of single puff intake, a display interface for single puff intake, a setting interface for a limit value of total intake, a display interface for total intake, and a display interface for residual amount of the limit value of total intake.

Preferably, the APP may comprise a maximum air pressure setting interface, a minimum air pressure setting interface, a setting interface for maximum output power of single puff, and a setting interface for minimum output power of single puff.

Preferably, the APP may comprise a wireless communication interface, a power on interface, a power off interface, a warning interface, and a battery indicator interface.

The disclosure provides another technical solution as follow. A method of controlling a vaporizing device capable of controlling administration amount comprises steps as follows.
(1) Setting parameters including:
   a maximum air pressure for enabling the vaporizing device to work,
   a minimum air pressure for enabling the vaporizing device to work,
   a limit value of single puff intake,
   a limit value of total intake,
   a maximum output power of single puff, and
   a minimum output power of single puff.
(2) By means of the microcontroller, determining whether the vaporizing device is in a power on state or not, if no, go to next step; if yes, go to step (5);
(3) Performing power on process;
(4) By means of the microcontroller, reading an air pressure parameter of the air pressure sensor;
(5) By means of the microcontroller, determining whether a smoking signal exists or not, if yes, go to next step; if no, go to step (17);
(6) By means of the microcontroller, determining whether the air pressure is less than the set minimum air pressure, if no, go to next step; if yes, go to step (17);
(7) By means of the microcontroller, determining whether the air pressure is greater than the set maximum air pressure, if no, go to next step; if yes, go to step (17);
(8) By means of the air flow calculating unit, calculating the air flow;
(9) By means of the power control unit, outputting corresponding power based on the air flow;
(10) Activating the timing unit to start timing;
(11) By means of the energy statistics unit, calculating energy;
(12) By means of the energy and vaporizing amount conversion unit, calculating corresponding value of aerosol intake from the energy;
(13) By means of the microcontroller, determining whether the single puff intake is greater than the limit value of single puff intake or not, if no, go to next step; if yes, go to step (17);
(14) Storing current intake parameter;
(15) Calculating the sum intake per unit time and determining whether the sum intake per unit time is greater than the limit value of total intake, if no, go to step (17); if yes, go to step (19);
(16) Storing current intake parameter;
(17) Entering a stand-by state;
(18) Returning to step (4);
(19) Entering a power off state.

### Advantages

The vaporizing device capable of controlling administration amount according to the disclosure may calculate energy consumption of the heating device, then calculate, control or restrict the administration amount of the vaporized aerosol or vapor, and thus may prevent the user from overusing the aerosol or vapor in single puff or in a unit time and avoid health hazards.

### BRIEF DESCRIPTION OF THE DRAWINGS

FIG. 1 is a cross-sectional view of a vaporizing device capable of controlling administration amount according to the disclosure;
FIG.2 is a functional block diagram of a circuit board of the disclosure;
FIG.3 is a diagram illustrating functions of the APP according to the disclosure;
FIG.4 is a flow chart of a controlling method according to the disclosure.

### DETAILED DESCRIPTION OF ILLUSTRATED EMBODIMENTS

In order to make purposes, technical solutions and advantages of the disclosure clearer, the disclosure will be further explained in detail with reference to drawings and embodiments described hereinafter. It should be understood that the specific embodiments described herein are only used to explain the present invention and are not intended to limit the present invention.

### Embodiments

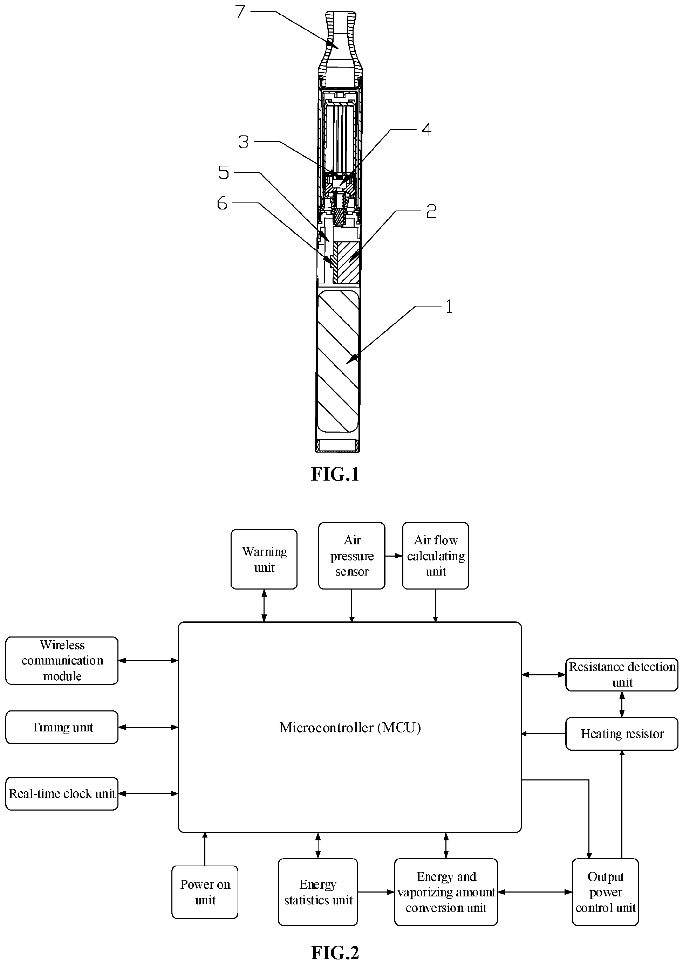

Referring to FIG. 1, a vaporizing device capable of controlling administration amount according to the disclosure comprises a battery 1, a control circuit board 2, a heating resistor 3, a vaporizing passage 4, and a mouth piece 7. The heating resistor 3 is disposed in the vaporizing passage 4, and the battery 1 supplies power for the heating resistor 3. The heating resistor 3 may be controlled by means of the control circuit board 2 to heat and vaporize liquid substances or paste-like substances, to generate aerosol or vapor for inhale. The user may inhale aerosol or vapor through the mouth piece 7. The vaporizing device capable of controlling administration amount according to the disclosure further comprises a detecting air passage 5 and an air pressure sensor 6 disposed beside the detecting air passage 5 to detect the suction pressure.

Referring to FIG.2, the control circuit board 2 may be arranged with a microcontroller MCU, an air flow calculating unit, an energy statistics unit, an energy and vaporizing amount conversion unit, and a power control unit, which are electrically connected with each other. The air flow calculating unit is configured to calculate the corresponding air flow quantity based on the modeling volume of the detecting air passage and the change of suction pressure. The power control unit is configured to control output power for the heating resistor based on the air flow quantity. The energy statistics unit is configured to calculate the energy output to the heating resistor based on the output power and the period of time. The energy and vaporizing amount conversion unit is configured to calculate the corresponding value of aerosol intake from the energy. The microcontroller MCU is configured to compare the value of aerosol intake with preset limit value of aerosol intake and then send corresponding control signal to the power control unit. On such basis, the power control unit is configured to control the output power to limit the aerosol intake.

The air pressure sensor 6 of the disclosure may be disposed beside the detecting air passage 5 to detect the suction pressure. According to Bernoulli equation applied for air: p+(1/2) ρv2=constant, wherein v=Q/S, it can be seen that the pressure and the flow quantity have a relationship of one-to-one correspondence. In the disclosure, by modeling calculation of the volume of the detecting air passage and the relationship of the pressure and the flow quantity, the flow quantity can be calculated from the suction pressure detected by means of the air pressure sensor. The air flow calculating unit of the disclosure is configured to calculate the flow quantity from the suction pressure detected by means of the air pressure sensor.

In the disclosure, the power control unit may be configured to control the output power output to the heating resistor based on the air flow quantity. That is, the greater the air flow quantity is (i.e., the greater the suction pressure is), the greater the output power is, and then the more aerosol (or vapor) will be generated. At the beginning of using, sufficient administration of aerosol or vapor may provide good user experience. However, to avoid overuse of medicine, vapor and the like which may be harmful to health, after a period of use, it is desired to control and limit the administration amount. The vaporizing process of liquid substances or paste-like substances such as drugs and E-cigarette liquid needs to absorb heat energy. The more heat energy is provided, the more aerosol will be generated. As the heat energy is converted from the electrical energy of the heating resistor, the control of aerosol intake can be achieved by controlling the power consumption of the vaporizing device.

In the disclosure, based on the output power and the period of time, the energy output to the heating resistor may be calculated by means of the energy statistics unit. As energy consumption for vaporizing is related with the amount of vaporized aerosol, the amount of the vaporized aerosol (i.e., the aerosol intake inhaled by the user) can be calculated from the energy consumption by modeling calculation.

In the disclosure, the corresponding value of aerosol intake may be calculated from the energy by means of the energy and vaporizing amount conversion unit, and then may be compared with the preset limit value of aerosol intake by means of the microcontroller, to determine whether it is close to or exceeds the limit value. Then, the corresponding control signal may be sent to the power control unit. After that, by means of the power control unit, the output power may be reduced or shut down, thereby preventing the user from overusing the medicine, the vapor, or the like. In this way, an automatic control for the aerosol intake of the user is achieved.

The limit value of aerosol intake set in the microcontroller MCU may comprise the limit value of single puff intake. When the value of single puff intake exceeds the limit value of single puff intake, the power control unit may shut down the output.

The limit value of aerosol intake set in the microcontroller may further comprise the limit value of total intake. When the sum value of aerosol intake per unit time exceeds the limit value of total intake, the power control unit may shut down the output.

Referring to FIG.2, the control circuit board may be further arranged with a resistance detection unit. The resistance detection unit is configured to detect a resistance change of the heating resistor and send the value of the resistance change to the microcontroller, and the microcontroller may control the power control unit based on the value of the resistance change.

The control circuit board may be further arranged with a real-time clock unit and a timing unit. Herein, the real-time clock unit may identify different timing during using of the vaporizing device, and the timing unit may calculate the period of using time.

The control circuit board may comprise a power on unit for turning on and activating the vaporizing device. The power on unit may be arranged in the form of a manual button, or in the form of activating by an automatic microphone, or in the form of being controlled by wireless communication of mobile phones.

The control circuit board of the disclosure may further comprise a warning unit electrically connected with the microcontroller. In the event of power on and off, stand-by time, the single puff intake exceeding the limit value, the total intake exceeding the limit value, overheat, overload, or dry-heating and the like, the warning unit may warn the user by a warning in the form of a sound, a light, a vibration, and the like.

The control circuit board may be further arranged with a wireless communication module electrically connected with the microcontroller. The wireless communication module may be in communication with the wireless communication module of the APP. By wireless communication, the APP may read the information of the vaporizing device and control operations of the vaporizing device such as parameter setting, power on and off.

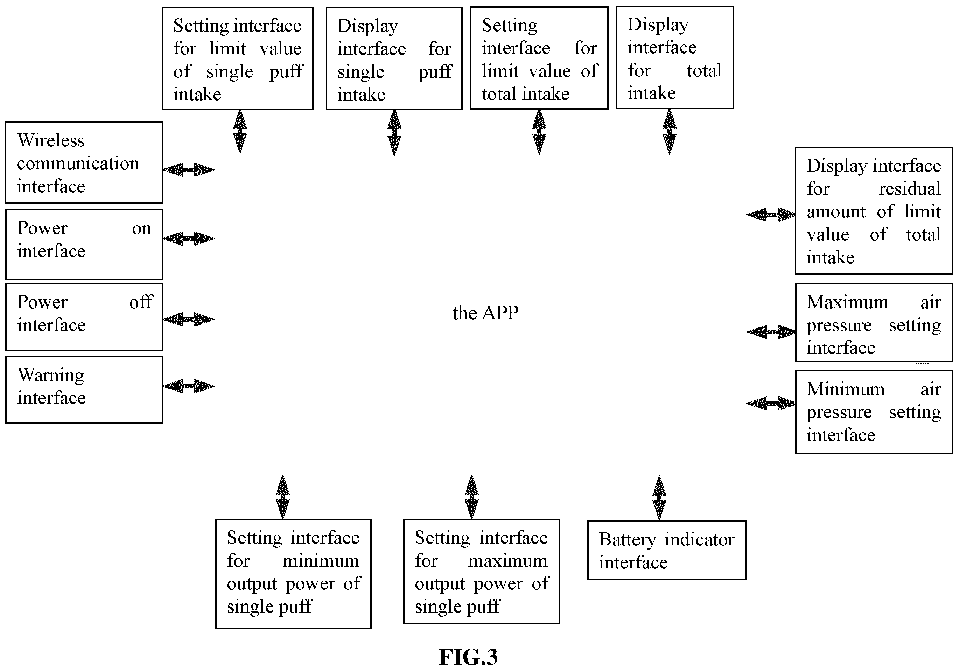

Referring to FIG.3, the APP may comprise a setting interface for limit value of single puff intake, a display interface for single puff intake, a setting interface for limit value of total intake, a display interface for total intake, and a display interface for residual amount of limit value of total intake.

The APP may comprise a maximum air pressure setting interface, a minimum air pressure setting interface, a setting interface for maximum output power of single puff, and a setting interface for minimum output power of single puff.

The APP may comprise a wireless communication interface, a power on interface, a power off interface, a warning interface, and a battery indicator interface.

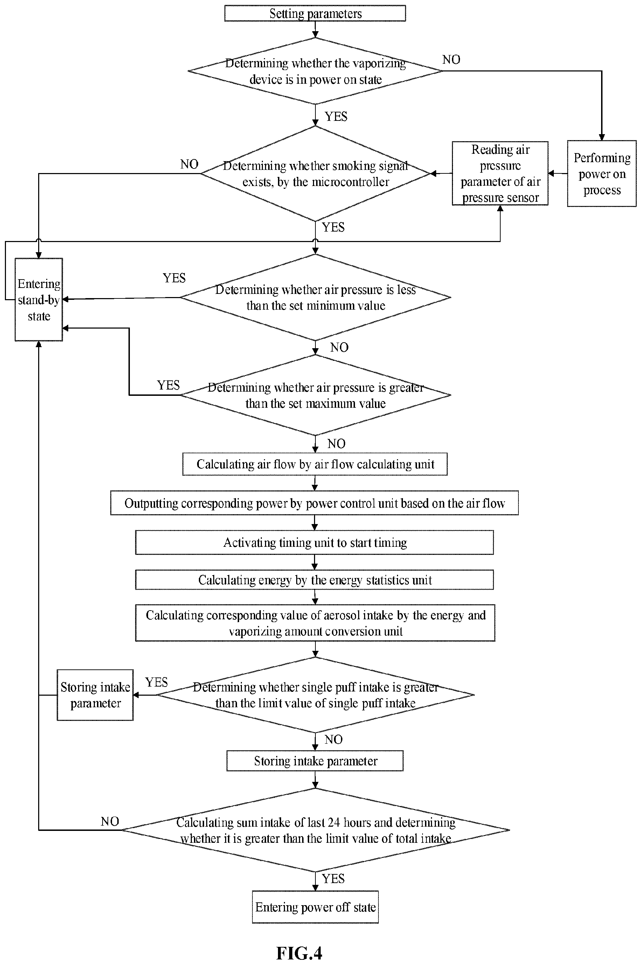

Referring to FIG.4, a method of controlling a vaporizing device capable of controlling administration amount comprises steps as follows.
(1) Setting parameters including:
   a maximum air pressure for enabling the vaporizing device to work,
   a minimum air pressure for enabling the vaporizing device to work,
   a limit value of single puff intake,
   a limit value of total intake,
   a maximum output power of single puff, and
   a minimum output power of single puff.
(2) By means of the microcontroller, determining whether the vaporizing device is in a power on state, if no, go to next step; if yes, go to step (5);
(3) Performing power on process, the vaporizing device being powered on by a manual button, an automatic microphone, or by the APP;
(4) Reading an air pressure parameter of the air pressure sensor by means of the microcontroller;
(5) By means of the microcontroller, determining whether a change of air pressure parameter reaches a set value or not, to determine whether a using signal exists, if yes, go to next step; if no, go to step (17);
(6) By means of the microcontroller, determining whether the air pressure is less than the set minimum air pressure, if no, go to next step; if yes, go to step (17);
(7) By means of the microcontroller, determining whether the air pressure is greater than the set maximum air pressure, if no, go to next step; if yes, go to step (17);
(8) By means of the air flow calculating unit, calculating the air flow based on the above air pressure parameters;
(9) By means of the power control unit, outputting corresponding power based on the air flow;
(10) Activating the timing unit to start timing;
(11) By means of the energy statistics unit, calculating energy, i.e., the product of the power and the time;
(12) By means of the energy and vaporizing amount conversion unit, calculating corresponding value of aerosol intake from the energy;
(13) By means of the microcontroller, determining whether the single puff intake is greater than the limit value of single puff intake, if no, go to next step; if yes, go to step (17);
(14) Storing current intake parameters;
(15) Calculating the sum intake per unit time (e.g., last 24 hours in the present embodiment) and determining whether it is greater than the limit value of total intake, if no, go to step (17); if yes, go to step (19);
(16) Storing current intake parameters;
(17) Entering a stand-by state;
(18) Returning to step (4);
(19) Entering a power off state.

### Industrial applicability

All the above are merely preferred embodiments of the disclosure. The present invention is intended to cover all modifications and equivalent arrangements those skilled in the art can make according to the technical essence of the present invention.

## Claims

1. A vaporizing device capable of controlling administration amount, comprising a battery, a control circuit board, a heating resistor, a vaporizing passage, and a mouth piece, wherein the heating resistor is disposed in the vaporizing passage and is controlled by means of the control circuit board to heat and vaporize liquid substances or paste-like substances to generate aerosol or vapor for inhale, wherein the vaporizing device further comprises a detecting air passage and an air pressure sensor disposed at the detecting air passage to detect suction pressure, the control circuit board is arranged with a microcontroller, an air flow calculating unit, an energy statistics unit, an energy and vaporizing amount conversion unit, and a power control unit, which are electrically connected with each other, wherein the air flow calculating unit is configured to calculate corresponding air flow quantity based on a modeling volume of the detecting air passage and a change of the suction pressure, wherein the power control unit is configured to control output power output to the heating resistor based on the air flow quantity, the energy statistics unit is configured to calculate energy output to the heating resistor based on the output power and time, the energy and vaporizing amount conversion unit is configured to calculate corresponding value of aerosol intake from the energy, the microcontroller is configured to compare the value of aerosol intake with preset limit value of aerosol intake and then send corresponding control signal to the power control unit, and the power control unit is configured to control the output power to limit aerosol intake.

2. The vaporizing device capable of controlling administration amount according to claim 1, wherein the limit value of aerosol intake set in the microcontroller comprises a limit value of single puff intake, and the power control unit is configured to shut down output if a value of single puff intake is greater than the limit value of single puff intake.

3. The vaporizing device capable of controlling administration amount according to claim 1, wherein the limit value of aerosol intake set in the microcontroller comprises a limit value of total intake, and the power control unit is configured to shut down output if a sum value of aerosol intake per unit time is greater than the limit value of total intake.

4. The vaporizing device capable of controlling administration amount according to claim 1, wherein the control circuit board further comprises a resistance detection unit configured to detect a resistance change of the heating resistor and send a value of the resistance change to the microcontroller, to allow the microcontroller to control the power control unit based on the value of the resistance change.

5. The vaporizing device capable of controlling administration amount according to claim 1, wherein the control circuit board is further arranged with a real-time clock unit and a timing unit.

6. The vaporizing device capable of controlling administration amount according to claim 1, wherein the control circuit board further comprises a power on unit for turning on and activating the vaporizing device.

7. The vaporizing device capable of controlling administration amount according to claim 1, wherein a warning unit electrically connected with the microcontroller is further provided.

8. The vaporizing device capable of controlling administration amount according to claim 1, wherein the control circuit board is further arranged with a wireless communication module electrically connected with the microcontroller, wherein the wireless communication module is in communication with a wireless communication module of APP, and the APP is configured to read information of the vaporizing device and control operations of the vaporizing device by wireless communication.

9. The vaporizing device capable of controlling administration amount according to claim 8, wherein the APP comprises a setting interface for a limit value of single puff intake, a display interface for single puff intake, a setting interface for a limit value of total intake, a display interface for total intake, and a display interface for residual amount of the limit value of total intake.

10. The vaporizing device capable of controlling administration amount according to claim 8, wherein the APP comprises a maximum air pressure setting interface, a minimum air pressure setting interface, a setting interface for maximum output power of single puff, and a setting interface for minimum output power of single puff.

11. The vaporizing device capable of controlling administration amount according to claim 8, wherein the APP comprises a wireless communication interface, a power on interface, a power off interface, a warning interface, and a battery indicator interface.

12. A method of controlling a vaporizing device capable of controlling administration amount, wherein the method comprises steps of:
(1) setting parameters including:
a maximum air pressure for enabling the vaporizing device to work,
a minimum air pressure for enabling the vaporizing device to work,
a limit value of single puff intake,
a limit value of total intake,
a maximum output power of single puff, and
a minimum output power of single puff;
(2) determining whether the vaporizing device is in a power on state or not by means of a microcontroller, if no, go to next step; if yes, go to step (5);
(3) performing power on process;
(4) reading an air pressure parameter of an air pressure sensor by means of the microcontroller;
(5) determining whether a smoking signal exists or not by means of the microcontroller, if yes, go to next step; if no, go to step (17);
(6) determining whether an air pressure is less than the set minimum air pressure or not by means of the microcontroller, if no, go to next step; if yes, go to step (17);
(7) determining whether the air pressure is greater than the set maximum air pressure or not by means of the microcontroller, if no, go to next step; if yes, go to step (17);
(8) calculating an air flow by means of an air flow calculating unit;
(9) outputting corresponding power by means of a power control unit based on the air flow;
(10) activating a timing unit to start timing;
(11) calculating energy by means of the energy statistics unit;
(12) calculating corresponding value of aerosol intake from the energy by means of the energy and vaporizing amount conversion unit;
(13) determining whether a single puff intake is greater than the limit value of single puff intake or not by means of the microcontroller, if no, go to next step; if yes, go to step (17);
(14) storing current intake parameter;
(15) calculating a sum intake per unit time and determining whether the sum intake per unit time is greater than the limit value of total intake, if no, go to step (17); if yes, go to step (19);
(16) storing current intake parameter;
(17) entering a stand-by state;
(18) returning to the step (4);
(19) entering a power off state.
